# EUROPEAN PATENT APPLICATION

(11) **EP 2 891 883 A1**
(43) Date of publication of application: **08.07.2015**
(21) Application number: 14195591.4
(22) Date of filing: 01.12.2014
(51) Int. Cl.: G01N 33/497, A61B 5/08, B60K 28/06

(54) **Gas concentration measurement apparatus and replacement notification method**

(30) Priority: 13.12.2013 JP 2013258283
(71) Applicant: Tanita Corporation, Tokyo 174-8630 (JP)
(72) Inventor: Honda, Daisuke, Tokyo, Tokyo 174-8630 (JP); Mochizuki, Kei, Tokyo, Tokyo 174-8630 (JP); Sagawa, Kiyoshi, Tokyo, Tokyo 174-8630 (JP)
(74) Representative: Grünecker Patent- und Rechtsanwälte PartG mbB

(57) **Abstract**

An alcohol sensor (204) outputs an electrical signal corresponding to the alcohol concentration. When an exposure mechanism (203) measures the alcohol concentration, the exposure mechanism (203) exposes the alcohol sensor (204) to the outside air. When the alcohol sensor (204) is exposed to the outside air, a CPU (205) calculates the alcohol concentration based on the electrical signal output by the alcohol sensor (204). The CPU (205) displays on a display a notification for replacement of the alcohol sensor when a total sum of a value corresponding to the duration that the alcohol sensor (204) has been exposed to the outside air exceeds a predetermined value.

## Description

### BACKGROUND

### Field of the Invention

The present invention relates to a gas concentration measurement apparatus and a replacement notification method.

### Background

Gas sensors provided in gas concentration measurement apparatuses, such as alcohol concentration measurement apparatuses, output an electrical signal corresponding to the gas concentration, as a result of a chemical reaction. Consequently, when a gas sensor is continuously utilized over a long period, the gas sensor deteriorates, and the response rate of the gas sensor decreases. That is to say, when deterioration of a gas sensor occurs, the gas detection accuracy decreases. Therefore, it is necessary for a user of a gas concentration measurement apparatus to replace the gas sensor provided in the gas concentration measurement apparatus before the gas detection accuracy decreases.

In Japanese Patent No. 4613184 there is disclosed a technique for issuing notification of a replacement timing when the usage frequency of the alcohol sensor exceeds a predetermined value.

### SUMMARY

The extent of deterioration of a gas sensor differs according to the utilization of the gas concentration measurement apparatus. Consequently, when a notification of a replacement timing is issued based on the usage frequency, in the manner of the technique disclosed in Japanese Patent No. 4613184. there is a possibility that a notification of a replacement timing is issued even though the gas sensor is still usable, or that a notification of the replacement timing is not issued even though the gas sensor is no longer usable.

A first aspect is a gas concentration measurement apparatus for performing a measurement of a gas concentration, the apparatus including: a gas sensor configured to output an electrical signal corresponding to the gas concentration in breath; a measuring part configured to measure the gas concentration based on an electrical signal output by the gas sensor; and a notification part configured to perform a notification for replacement of the gas sensor in at least one of a case (a) where a total sum of value corresponding to a duration that the gas sensor has been exposed to outside air exceeds a predetermined value and a case (b) where a total sum of value corresponding to the gas concentration measured by the measuring part exceeds a predetermined value.

A second aspect is a notification method of a gas concentration measurement apparatus, wherein the gas concentration measurement apparatus includes: a gas sensor configured to output an electrical signal corresponding to a gas concentration in breath; and a measuring part configured to measure the gas concentration based on the electrical signal from the gas sensor, wherein the method includes: determining whether or not to perform a notification for replacement of the gas sensor; and performing the notification for replacement of the gas sensor, wherein the determining includes at least one of (a) determining whether or not a total sum of value corresponding to a duration that the gas sensor has been exposed to outside air exceeds a predetermined value and (b) determining whether or not a total sum of value corresponding to the gas concentration measured by the measuring part exceeds a predetermined value, and wherein the performing includes performing a notification in at least one of a case (a) where a total sum of value corresponding to a duration that the gas sensor has been exposed to outside air exceeds a predetermined value and a case (b) where a total sum of value corresponding to the gas concentration measured by the measuring part exceeds a predetermined value.

According to at least one aspect among the aspects described above, a gas concentration measurement apparatus is able to provide notification for replacement of a gas sensor according to the actual deterioration of the gas sensor, based on the duration of exposure of the gas sensor, the detected gas concentration, and the like.

### BRIEF DESCRIPTION OF THE DRAWINGS

FIG. 1 is an external view of an alcohol concentration measurement apparatus according to one embodiment.
FIG. 2 is a schematic view showing an internal configuration of the alcohol concentration measurement apparatus according to one embodiment.
FIG. 3 is a schematic block diagram showing a software configuration of the alcohol concentration measurement apparatus according to one embodiment.
FIG. 4 is a first flowchart showing operation of the alcohol concentration measurement apparatus according to one embodiment.
FIG. 5 is a second flowchart showing operation of the alcohol concentration measurement apparatus according to one embodiment.
FIG. 6A is a drawing showing a display example of the extent of deterioration of an alcohol sensor.
FIG. 6B is a drawing showing a display example of the extent of deterioration of an alcohol sensor.
FIG. 6C is a drawing showing a display example of the extent of deterioration of an alcohol sensor.

### BRIEF DESCRIPTION OF THE REFERENCE NUMERALS

- 1: ALCOHOL CONCENTRATION MEASUREMENT APPARATUS
- 101: HOUSING
- 102: DISPLAY
- 103: OPERATION BUTTON
- 104: MOUTHPIECE
- 105: SOCKET
- 201: BREATH PASSAGE
- 202: PRESSURE SENSOR
- 203: ELECTROMAGNETIC VALVE
- 204: ALCOHOL SENSOR
- 205: CPU
- 206: MAIN STORAGE DEVICE
- 207: AUXILIARY STORAGE DEVICE
- 208: INTERFACE
- 301: REPLACEMENT DETERMINATION PART
- 302: EXPOSURE INSTRUCTING PART
- 303: ALCOHOL CONCENTRATION MEASURING PART
- 304: ISOLATION INSTRUCTING PART
- 305: TIMING PART
- 306: UPDATING PART

### DESCRIPTION OF THE EMBODIMENTS

Hereunder, an embodiment is described in detail with reference to the drawings.

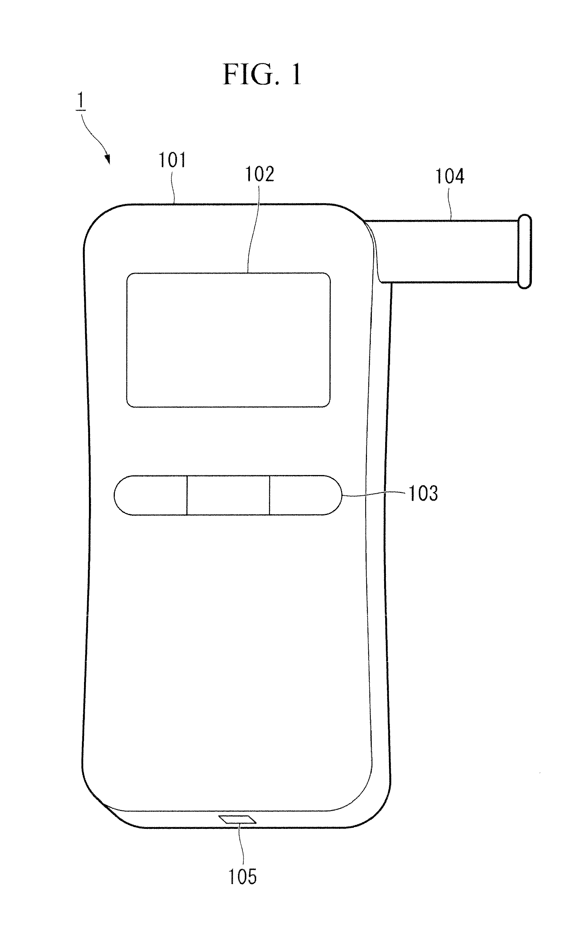

FIG. 1 is an external view of an alcohol concentration measurement apparatus 1 according to one embodiment.

The alcohol concentration measurement apparatus 1 includes a housing 101, a display 102, an operation button 103, a mouthpiece 104, and a socket 105. In the present embodiment, the alcohol concentration measurement apparatus 1 is an example of a gas concentration measurement apparatus. Furthermore, in the present embodiment, the gas representing the measurement target is an alcohol.

The housing 101 forms a casing of the alcohol concentration measurement apparatus 1.

The display 102 displays a measurement result of the alcohol concentration according to the alcohol concentration measurement apparatus 1, or the like.

The operation button 103 is an input device for operating the alcohol concentration measurement apparatus 1.

The mouthpiece 104 is a delivery port (blow port) for a breath of a test subject.

The socket 105 is a receiving port for a plug that connects the alcohol concentration measurement apparatus I with an external device.

The test subject starts up the alcohol concentration measurement apparatus 1 by operating the operation button 103. Then, the alcohol concentration measurement apparatus 1 performs a measurement of the alcohol concentration in breath once the test subject delivers a breath into the alcohol concentration measurement apparatus 1 via the mouthpiece 104. Once the alcohol concentration measurement apparatus 1 completes the alcohol concentration measurement, the alcohol concentration measurement apparatus 1 displays the measurement result on the display 102 or an external device connected via the socket 105. Furthermore, the alcohol concentration measurement apparatus 1 displays a notification for replacement of the alcohol sensor 204 on the display 102. That is to say, in the present embodiment, the display 102 is an example of a notification part.

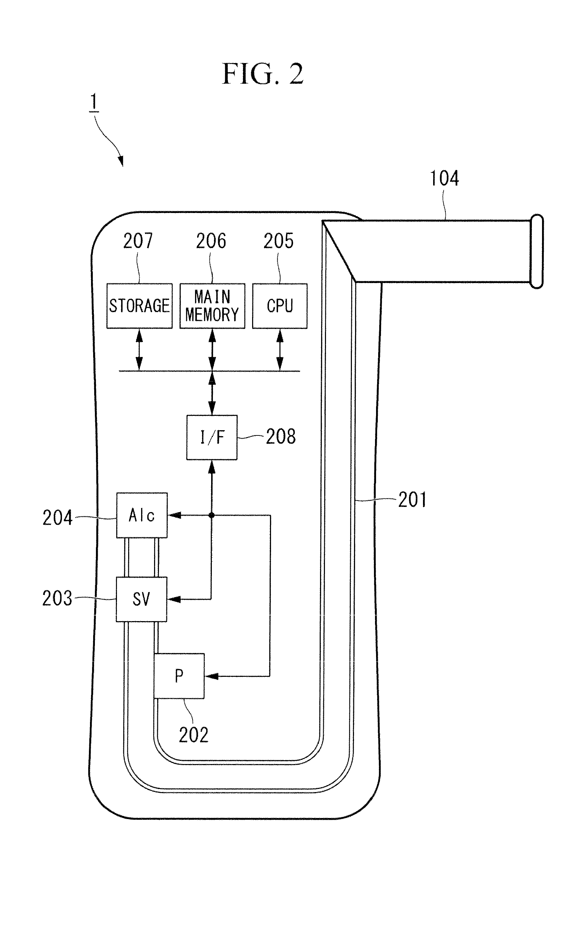

FIG. 2 is a schematic view showing an internal configuration of the alcohol concentration measurement apparatus 1 according to one embodiment.

The alcohol concentration measurement apparatus 1 includes; a breath passage 201, a pressure sensor 202, an electromagnetic valve 203, an alcohol sensor 204, a CPU 205, a main storage device 206 (main memory), an auxiliary storage device 207 (storage), and an interface 208 (I/F).

The breath passage 201 is a piping that guides a breath delivered in via the mouthpiece 104 to the pressure sensor 202 and the alcohol sensor 204.

The pressure sensor 202 converts the pressure within the breath passage 201 into an electrical signal, and outputs the electrical signal to the interface 208.

The electromagnetic valve 203 is provided such that the electromagnetic valve 203 can open and close the breath passage 201 between the alcohol sensor 204 and the mouthpiece 104. The alcohol sensor 204 is isolated from the outside air due to the closing of the electromagnetic valve 203. The alcohol sensor 204 is exposed to the outside air via the breath passage 201 due to the opening of the electromagnetic valve 203. In the present embodiment, the electromagnetic valve 203 is an example of an exposure mechanism.

The alcohol sensor 204 converts the alcohol concentration of a breath that flows through the breath passage 201 into an electrical signal, and outputs the electrical signal to the interface 208. In the present embodiment, the alcohol sensor 204 is an example of a gas sensor. The alcohol sensor 204 according to the present embodiment is a fuel cell alcohol sensor. Specifically, the alcohol sensor 204 is provided with a polymer membrane that is permeable to hydrogen ions, and a platinum catalyst. The platinum catalyst separates hydrogen ions from the alcohol in breath, and the alcohol sensor 204 generates electricity as a result of the hydrogen ions reacting with the oxygen in air via the polymer membrane. Consequently, the higher the alcohol concentration in breath, the greater the quantity of hydrogen ions separated by the platinum catalyst, and thus the greater the electrical current generated by the alcohol sensor 204.

On the other hand, when the alcohol sensor 204 is continuously utilized and the total time that the alcohol sensor 204 is exposed to the outside air becomes long, the activity of the platinum catalyst gradually decreases. Consequently, the activation of oxygen and hydrogen in the alcohol sensor 204 becomes low. That is to say, deterioration of the alcohol sensor 204 occurs. Furthermore, when the alcohol sensor 204 is exposed to a breath that contains alcohol, water is generated due to a reaction between hydrogen and oxygen. Consequently, if the alcohol sensor 204 is exposed to a breath with a high alcohol concentration, the response becomes slow compared to a case where the alcohol sensor 204 is exposed to a breath with a low alcohol concentration. That is to say, deterioration of the alcohol sensor 204 occurs.

The CPU 205 is started up by an operation of the operation button 103. The CPU 205 reads a program from the auxiliary storage device 207 and transfers the program to the main storage device 206, and a predetermined process is executed according to the program. Specifically, the CPU 205 performs according to the program; a measurement process of the alcohol concentration in breath, and a determination process for replacement of the alcohol sensor 204. That is to say, in the present embodiment, the CPU 205 is an example of a measuring part.

The main storage device 206 is a storage device directly accessed by the CPU 205.

The auxiliary storage device 207 stores a program for making the CPU 205 execute an alcohol measurement process and a replacement determination process of the alcohol sensor 204. Furthermore, a usage start date and time of the alcohol sensor 204, and a total usage time of the alcohol sensor 204 are stored in the auxiliary storage device 207. The usage start date and time of the alcohol sensor 204 is the date and time that the alcohol concentration measurement apparatus 1 was shipped, and is recorded in the auxiliary storage device 207 by the vendor of the alcohol concentration measurement apparatus 1.

The interface 208 outputs signals that are output by peripheral devices to the CPU 205, and furthermore, outputs instructions from the CPU 205 to the peripheral devices. The peripheral devices in the present embodiment are external devices that are connected via the pressure sensor 202, the electromagnetic valve 203, the alcohol sensor 204, the display 102, and the socket 105.

In the present embodiment, the CPU 205, the main storage device 206, the auxiliary storage device 207, and the interface 208 are an example of a computer.

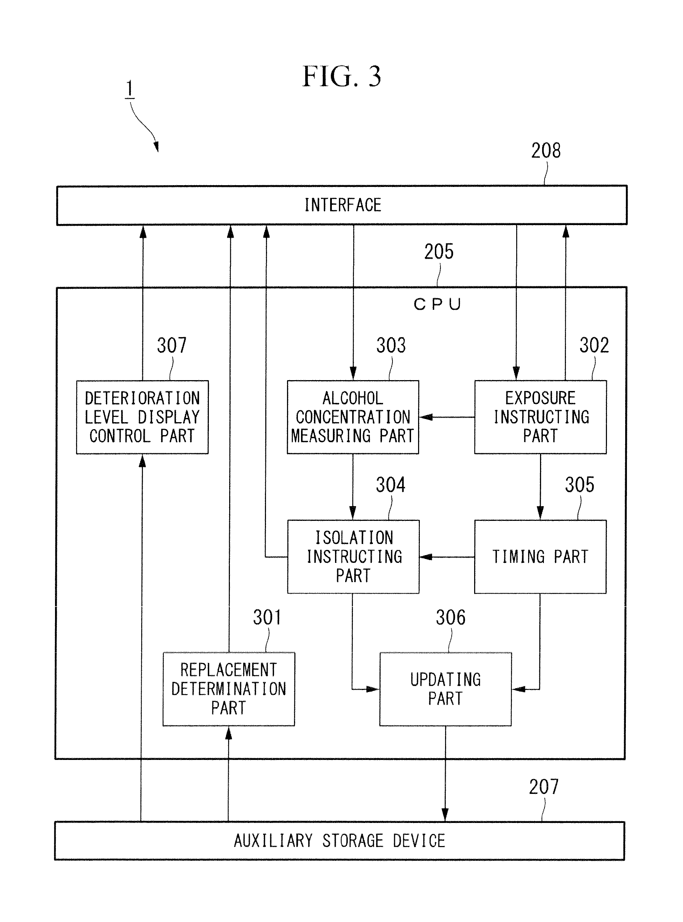

FIG. 3 is a schematic block diagram showing a software configuration of the alcohol concentration measurement apparatus 1 according to one embodiment.

The CPU 205 is provided with, as a result of executing the program stored in the auxiliary storage device 207, a replacement determination part 301, an exposure instructing part 302, an alcohol concentration measuring part 303, an isolation instructing part 304, a timing part 305, an updating part (a history updating part) 306, and a deterioration level display control part 307.

The replacement determination part 301 determines whether or not the alcohol sensor 204 should be replaced based on the usage start date and time of the alcohol sensor 204, and the total usage time of the alcohol sensor 204 recorded in the auxiliary storage device 207. Specifically, the replacement determination part 301 determines that the alcohol sensor 204 should be replaced when the time period from the usage start date and time of the alcohol sensor 204 to the current time, that is to say, the usage time period of the alcohol sensor 204, exceeds a predetermined threshold (for example, one year). Furthermore, the replacement determination part 301 determines that the alcohol sensor 204 should be replaced when the total usage time of the alcohol sensor 204, that is to say, a total sum of a value corresponding to the duration that the alcohol sensor 204 has been exposed to the outside air, exceeds a predetermined value.

The exposure instructing part 302 reads out the pressure within the breath passage 201 from the pressure sensor 202 via the interface 208. The exposure instructing part 302 determines whether or not a breath is being delivered into the breath passage 201 on the basis of the pressure within the breath passage 201. The exposure instructing part 302 outputs to the electromagnetic valve 203 via the interface 208, an instruction to open the breath passage 201 when a breath has been delivered into the breath passage 201 for a predetermined time (for example, 5 sec). The alcohol sensor 204 is exposed to the outside air as a result of the electromagnetic valve 203 opening according to the instruction from the exposure instructing part 302.

The alcohol concentration measuring part 303 acquires from the alcohol sensor 204 via the interface 208, a signal according to the alcohol concentration. The alcohol concentration measuring part 303 determines a peak value for the signal acquired from the alcohol sensor 204. The alcohol concentration measuring part 303 integrates the value indicated by the signal until the value of the signal acquired from the alcohol sensor 204 becomes less than or equal to a predetermined threshold (for example, 20% of the peak value). The alcohol concentration measuring part 303 measures the alcohol concentration in breath on the basis of the peak value and the integrated value of the signal.

In the present embodiment, the time associated with an alcohol concentration measurement can be kept to approximately 5 sec by using 20% of the peak value as the threshold. On the other hand, instead of using a threshold representing a relative value with respect to the peak value, an absolute value can also be applied as the threshold. In this case, the time taken for the signal value acquired from the alcohol sensor 204 to become less than or equal to the threshold, monotonically increases with the alcohol concentration.

Once the alcohol concentration measuring part 303 completes an alcohol concentration measurement, and after elapse of a recovery time corresponding to the measurement result, the isolation instructing part 304 outputs to the electromagnetic valve 203 via the interface 208, an instruction to close the breath passage 201. This is carried out in order to expose the alcohol sensor 204 to the outside air until the alcohol leaves the platinum catalyst of the alcohol sensor 204. The recovery time is a monotonically non-decreasing time with respect to the detected alcohol concentration. This is because the higher the alcohol concentration, the longer the time taken for the alcohol to leave the alcohol sensor 204. The alcohol sensor 204 is isolated from the outside air as a result of the electromagnetic valve 203 closing according to the instruction from the isolation instructing part 304.

The timing part 305 measures the usage time of the alcohol sensor 204. The usage time of the alcohol sensor 204 is the duration from the time at which the exposure instructing part 302 determines that a breath is being delivered into the breath passage 201, to the time at which the isolation instructing part 304 outputs to the electromagnetic valve 203 an instruction to close the breath passage 201.

The updating part 306 adds the time that is measured by the timing part 305 to the total usage time of the alcohol sensor 204 stored in the auxiliary storage device 207.

The deterioration level display control part 307 reads out the usage start date and time, and the total usage time from the auxiliary storage device 207, and displays on the display 102 the extent of deterioration of the alcohol sensor 204 calculated based on the usage start date and time and the total usage time.

Next, an operation of the alcohol concentration measurement apparatus 1 according to the present embodiment is described.

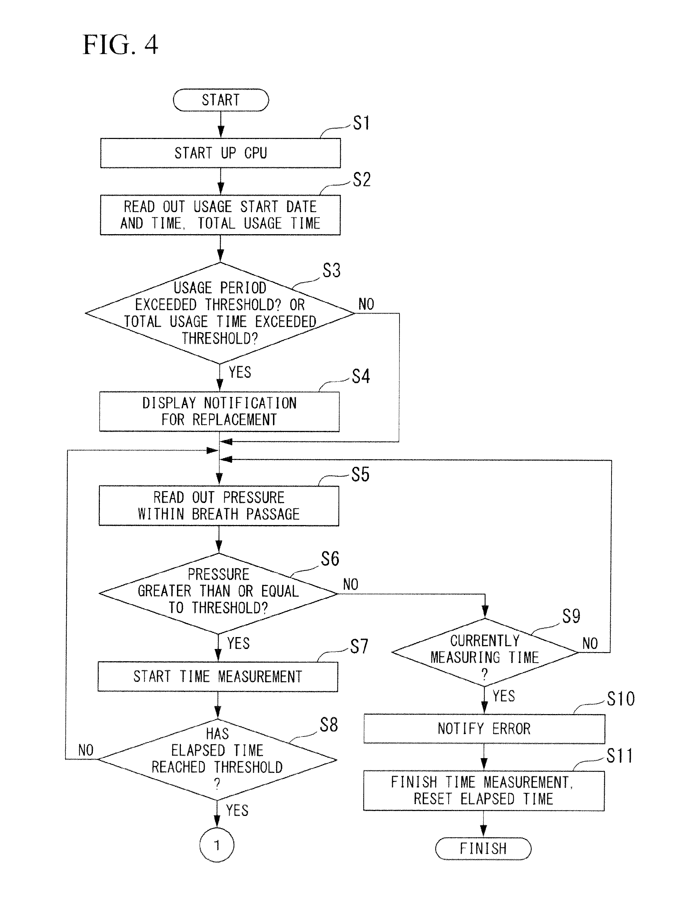

FIG. 4 is a first flow chart showing operation of the alcohol concentration measurement apparatus 1 according to one embodiment.

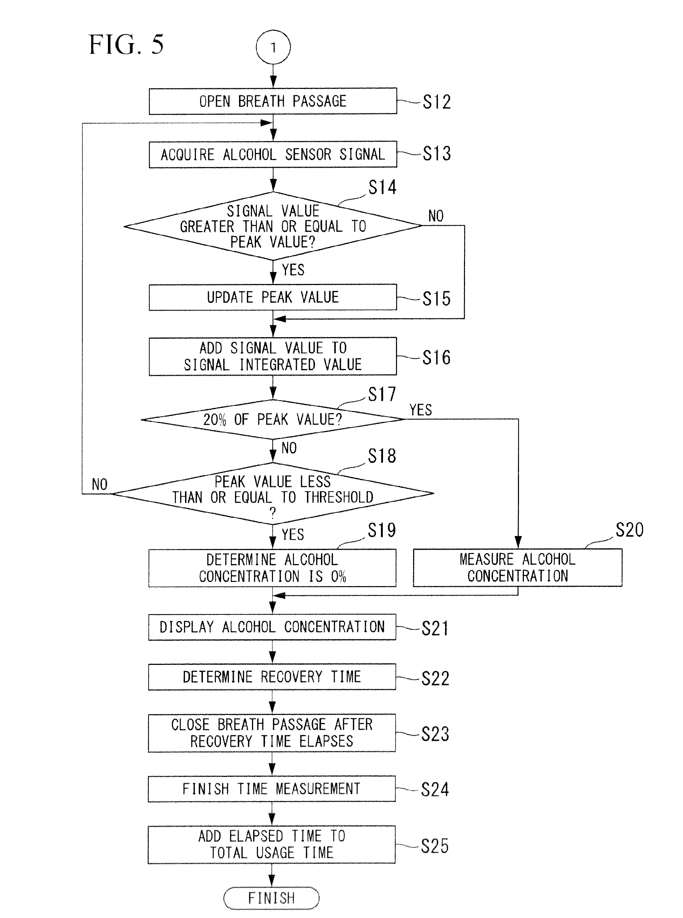

FIG. 5 is a second flow chart showing operation of the alcohol concentration measurement apparatus 1 according to one embodiment.

The CPU 205 starts up as a result of a test subject depressing the operation button 103 (step S1). Once the CPU 205 starts up, the CPU 205 executes the program stored in the auxiliary storage device 207.

When the CPU 205 executes the program, the replacement determination part 301 reads out the usage start date and time of the alcohol sensor 204, and the total usage time of the alcohol sensor 204, which are stored in the auxiliary storage device 207 (step S2). The replacement determination part 301 determines on the basis of the usage start date and time, and the total usage time that are read out, whether or not the usage period of the alcohol sensor 204 has exceeded a predetermined threshold, or whether or not the total usage time of the alcohol sensor 204 has exceeded a predetermined threshold (step S3).

If the replacement determination part 301 determines that the usage period of the alcohol sensor 204 has exceeded the predetermined threshold, or the replacement determination part 301 determines that the total usage time of the alcohol sensor 204 has exceeded the predetermined threshold (step S3: YES), the replacement determination part 301 determines that the alcohol sensor 204 should be replaced. When the replacement determination part 301 determines that the alcohol sensor 204 should be replaced, the replacement determination part 301 displays on the display 102 via the interface 208, a notification for replacement of the alcohol sensor 204 (step S4). On the other hand, if the replacement determination part 301 determines that the usage period of the alcohol sensor 204 has not exceeded the predetermined threshold, and the replacement determination part 301 determines that the total usage time of the alcohol sensor 204 has not exceeded the predetermined threshold (step S3: NO), the replacement determination part 301 determines that replacement of the alcohol sensor 204 is not required.

When the display 102 displays a notification for replacement, or the replacement determination part 301 determines that replacement of the alcohol sensor 204 is not required, the exposure instructing part 302 reads out the pressure within the breath passage 201 from the pressure sensor 202 via the interface 208 (step S5). Then, the exposure instructing part 302 determines whether or not the pressure within the breath passage 201 is greater than or equal to a predetermined threshold (step S6).

If the exposure instructing part 302 determines that the pressure within the breath passage 201 is greater than or equal to the threshold (step S6: YES), the exposure instructing part 302 determines that a breath is being delivered into the breath passage 201 via the mouthpiece 104. When the exposure instructing part 302 determines that a breath is being delivered into the breath passage 201, the timing part 305 starts measuring an elapsed time from the current time (step S7). If the timing part 305 is already measuring an elapsed time, the timing part 305 continues the measurement.

Next, the exposure instructing part 302 determines whether or not the elapsed time being measured by the timing part 305 has reached a predetermined threshold (step S8). If the exposure instructing part 302 determines that the elapsed time has not reached the threshold (step S8: NO), the process returns to step S5, and the determination of the pressure within the breath passage 201 is continued.

On the other hand, in step S6, if the exposure instructing part 302 determines that the pressure within the breath passage 201 is less than the threshold (step S6: NO), the exposure instructing part 302 determines whether or not the timing part 305 is measuring an elapsed time (step S9). If the pressure within the breath passage 201 is less than the threshold and the timing part 305 is not measuring an elapsed time (step S9: NO), the exposure instructing part 302 determines that a breath is not being delivered in by the test subject. If the exposure instructing part 302 determines that a breath is not being delivered in, the process returns to step S5, and continues determination of a breath being delivered in.

On the other hand, if the pressure within the breath passage 201 is less than the threshold, and the timing part 305 is measuring an elapsed time (step S9: YES), the exposure instructing part 302 determines that the delivery of a breath by the test subject has been interrupted. If the exposure instructing part 302 determines that the delivery of the breath has been interrupted, the exposure instructing part 302 displays on the display 102 via the interface 208, a notification of an error due to the interruption of the delivery of the breath (step S10). Furthermore, the timing part 305 completes the measuring of the elapsed time, and resets the elapsed time (step S11).

In step S8, if the exposure instructing part 302 determines that the elapsed time has reached the threshold (step S8: YES), the exposure instructing part 302 determines that a sufficient amount of breath for a measurement has been delivered into the breath passage 201. If the exposure instructing part 302 determines that a sufficient amount of breath for a measurement has been delivered in, the exposure instructing part 302 outputs to the electromagnetic valve 203 via the interface 208, an instruction to open the breath passage 201 (step S12).

When the exposure instructing part 302 outputs the instruction, the alcohol concentration measuring part 303 acquires from the alcohol sensor 204 via the interface 208, a signal corresponding to the alcohol concentration (step S13). The alcohol concentration measuring part 303 determines whether or not the value of the signal acquired from the alcohol sensor 204 is larger than the peak value of the signal stored in the main storage device 206 (step S14). If the alcohol concentration measuring part 303 determines that the value of the acquired signal is larger than the peak value stored in the main storage device 206 (step S14: YES), the alcohol concentration measuring part 303 updates the peak value stored in the main storage device 206 (step S15). On the other hand, if the alcohol concentration measuring part 303 determines that the value of the acquired signal is less than or equal to the maximum value stored in the main storage device 206 (step S14: NO), the alcohol concentration measuring part 303 does not update the maximum value stored in the main storage device 206. Then, the alcohol concentration measuring part 303 adds the value of the signal acquired from the alcohol sensor 204 to a signal integrated value stored in the main storage device 206 (step S16).

Next, the alcohol concentration measuring part 303 determines whether or not the value of the acquired signal is less than or equal to 20% of the peak value stored in the main storage device 206 (step S17). If the alcohol concentration measuring part 303 determines that the value of the acquired signal is larger than 20% of the peak value (step S17: NO), the alcohol concentration measuring part 303 determines whether or not a peak value measured within a predetermined time (for example, 1 sec) from when signal acquisition is started is less than or equal to a predetermined threshold (step S18).

If the alcohol concentration measuring part 303 determines that the value of the acquired signal is larger than 20% of the peak value, and the peak value is larger than the threshold (step S18: NO), the process returns to step S13 and signal acquisition from the alcohol sensor 204 is continued. On the other hand, if the alcohol concentration measuring part 303 determines that the value of the acquired signal is larger than 20% of the peak value, and the peak value is smaller than the threshold (step S18: YES), the alcohol concentration measuring part 303 determines that alcohol is not contained in the breath (step S 19). That is to say, the alcohol concentration measuring part 303 determines that the alcohol concentration contained in the breath is 0%.

On the other hand, in step S17, if the alcohol concentration measuring part 303 determines that the value of the acquired signal is less than or equal to the predetermined threshold (step S17: YES), the alcohol concentration measuring part 303 calculates the alcohol concentration that is contained in the breath on the basis of the peak value and the signal integrated value stored in the main storage device 206 (step S20).

When the alcohol concentration measuring part 303 measures the alcohol concentration in step S 19 or in step S20, the alcohol concentration measuring part 303 displays the alcohol concentration on the display 102 via the interface 208 (step S21).

Next, the isolation instructing part 304 determines the recovery time on the basis of the concentration measured by the alcohol concentration measuring part 303 (step S22). In the present embodiment, the isolation instructing part 304 determines the recovery time according to a table that associates the concentration and the recovery time. In the table, the recovery time is a monotonically non-decreasing time with respect to the detected alcohol concentration. When the isolation instructing part 304 determines the recovery time, the isolation instructing part 304 outputs to the electromagnetic valve 203 via the interface 208, an instruction to close the breath passage 201 after elapse of the recovery time (step S23).

When the isolation instructing part 304 outputs the instruction, the timing part 305 completes measurement of the elapsed time (step S24). Then, the updating part 306 adds the time measured by the timing part 305 to the total usage time of the alcohol sensor 204 stored in the auxiliary storage device 207 (step S25). Once the updating part 306 updates the total usage time, the timing part 305 resets the measured elapsed time. Consequently, the alcohol concentration measurement apparatus 1 completes processing, and stops supplying power to the CPU 205.

The replacement determination part 301 determines, according to the processing described above, whether or not the alcohol sensor should be replaced on the basis of the usage period of the alcohol sensor 204 and the total usage time of the alcohol sensor 204. The total usage time of the alcohol sensor 204 is the total sum of a value corresponding to the duration that the alcohol sensor 204 has been exposed to the outside air. Therefore, the alcohol concentration measurement apparatus 1 is able to perform a notification for replacement when the activity of the platinum catalyst has decreased due to the exposure of the alcohol sensor 204 to the outside air.

Furthermore, the time taken for the value of the electrical signal output by the alcohol sensor 204 to become less than or equal to the predetermined threshold, and the waiting time, are monotonically non-decreasing times with respect to the alcohol concentration contained in the breath. That is to say, the total usage time of the alcohol sensor 204 is a total sum of a value corresponding to the alcohol concentration. Therefore, the alcohol concentration measurement apparatus 1 is able to perform a notification for replacement when deterioration of the alcohol sensor 204 occurs due to the water generated from reaction of the hydrogen separated from the alcohol, and components contained in the breath, such as S (sulfur).

While the alcohol concentration measurement apparatus 1 is starting up, the alcohol concentration measurement apparatus 1 displays the extent of deterioration of the alcohol sensor 204 on the display 102.

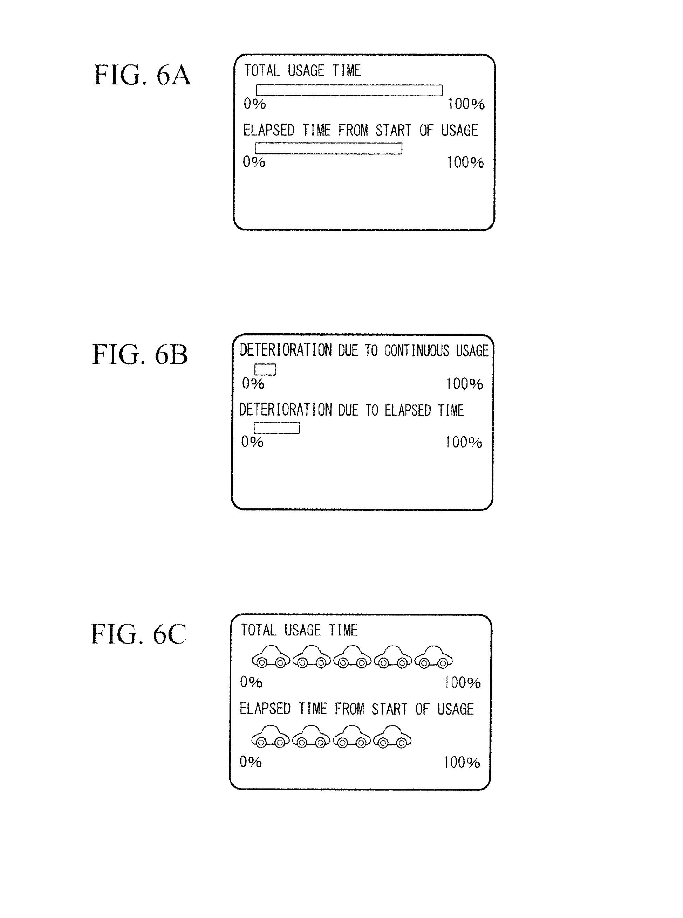

FIG. 6A to FIG. 6C are drawings showing a display example of the extent of deterioration of the alcohol sensor.

The deterioration level display control part 307 reads out the usage start date and time and the total usage time from the auxiliary storage device 207, and calculates the extent of deterioration of the alcohol sensor 204 based on the usage start date and time and the total usage time. Specitically, the deterioration level display control part 307 calculates, as the extent of deterioration, a fraction obtained by dividing the total usage time by the usage time threshold (a fraction obtained by dividing the total sum of a value corresponding to the duration that the alcohol sensor has been exposed to the outside air by a predetermined value at which a notification for replacement of the alcohol sensor is performed), and a fraction obtained by dividing the elapsed time from the usage start date and time by an elapsed time threshold. The fraction obtained by dividing the total usage time by the usage time threshold is equivalent to a fraction obtained by dividing the total sum of a value corresponding to the alcohol concentration measured by the alcohol concentration measuring part 303 by a predetermined value at which a notification for replacement of the alcohol sensor 204 is performed. Further, the deterioration level display control part 307 displays the calculated extent of deterioration on the display 102 via the interface 208.

A display example of the extent of deterioration includes a method as shown in FIG. 6A and FIG. 6B, in which the fraction with respect to the threshold of the elapsed time from the usage start date and time, and the fraction with respect to the threshold of the total usage time, are respectively displayed as bars. In the example shown in FIG. 6A, the length of the bar is longest when there is no deterioration of the alcohol sensor 204. The length of the bar is shortest when the alcohol sensor 204 should be replaced. In the example shown in FIG. 6B, the length of the bar is shortest when there is no deterioration of the alcohol sensor 204. The length of the bar is longest when the alcohol sensor 204 should be replaced.

At this time, the color of the bar may be changed according to the extent of deterioration of the alcohol sensor 204. For example, the color of the bar may be changed such that the color is green when the fraction obtained by dividing the total usage time of the alcohol sensor 204 by the usage time threshold is less than 25%, yellow when the fraction is 25% to 75%, and red when the fraction is greater than or equal to 75%. The same applies to the time elapsed from the usage start date and time.

Furthermore, the length of the bar may take a linear value with respect to the elapsed time or the total usage time (i.e., the indicated value of the bar can be substantially linearly changed with respect to the elapsed time or the total usage time), and the length may also take a logarithmic value (i.e., the indicated value of the bar can be substantially logarithmically changed with respect to the elapsed time or the total usage time). The necessity for replacement can be emphasized by the length of the bar taking a logarithmic value.

Another display example includes a method as shown in FIG. 6C, in which the elapsed time from the usage start date and time, and the total usage time, are respectively represented by a number of symbols or icons. In the example shown in FIG. 6C, the number of symbols or icons is greatest when there is no deterioration of the alcohol sensor 204. The number of symbols or icons becomes smallest (zero) when the alcohol sensor 204 should be replaced.

Furthermore, the deterioration level display control part 307 may simply display on the display 102 a numerical value indicating the fraction with respect to the threshold of the elapsed time from the usage start date and time, and a numerical value indicating the fraction with respect to the threshold of the total usage time.

The foregoing has described in detail one embodiment with reference to the drawings. However, specific configurations are in no way limited to those mentioned above, and various design changes, and the like, are possible.

For example, in the present embodiment, a case is described where the gas concentration measurement apparatus is implemented by the alcohol concentration measurement apparatus 1. However, it is in no way limited to this. For example, the functions of the gas concentration measurement apparatus according to the present invention may be applied to other gas concentration measurement apparatuses. Examples of other gas concentration measurement apparatuses include a body fat burn amount measuring device that measures the extent to which body fat is burned by detecting the acetone concentration in breath, and an oral odor detection device that measures the concentration of gases that cause oral odor, such as methylmercaptan. Furthermore, in the present embodiment, a case is described where the alcohol sensor 204 is a fuel cell alcohol sensor. However, it is in no way limited to this. For example, the alcohol concentration measurement apparatus 1 may be provided with a semiconductor alcohol sensor.

Furthermore, in the present embodiment, a case is described where timing part 305 measures the duration from the time at which a breath is delivered into the breath passage 201 to the time at which the recovery time has elapsed after measurement of the alcohol concentration, and the determination of replacement is made on the basis of the total sum of the duration. However, it is in no way limited to this. For example, the updating part 306 may measure the time between the exposure instructing part 302 outputting an instruction to open the breath passage 201 and the isolation instructing part 304 outputting an instruction to close the breath passage 201.

Furthermore, the usage time measured by the timing part 305 is also an example of a value corresponding to the alcohol concentration measured by the alcohol concentration measuring part 303. On the other hand, the timing part 305 may measure the recovery time determined by the isolation determination part, or the time taken for the signal value output by the alcohol sensor 204 to become less than or equal to the predetermined threshold, as a value that corresponds to the alcohol concentration measured by the alcohol concentration measuring part 303.

Moreover, the updating part 306 may add the duration from the time at which a breath is delivered into the breath passage 201 to the time at which time measurement is stopped as a result of an error, to the total usage time of the alcohol sensor 204.

In the present embodiment, a case is described where the replacement determination part 301 performs the determination of replacement on the basis of the usage time of the alcohol sensor 204, as an example of a value corresponding to the duration that the alcohol sensor 204 has been exposed to the outside air. However, it is in no way limited to this. For example, the replacement determination part 301 may perform the determination of replacement using a predetermined parameter that is monotonically non-decreasing with respect to the duration that the alcohol sensor 204 has been exposed to the outside air. In one example, a fraction obtained based on a table that associates the concentration and the recovery time (e.g., a time till the gas (alcohol) leaves the alcohol sensor 204) can be displayed on the deterioration level display control part 307.

Furthermore, in the present embodiment, a case is described in which the alcohol sensor 204 is a fuel cell type. However, it is in no way limited to this. A semiconductor type, or other types of alcohol sensors may also be used.

Moreover, in the present embodiment, a case is described in which the extent of deterioration of the alcohol sensor 204 is displayed on the display 102. However, it is in no way limited to this. For example, in another embodiment, the extent of deterioration of the alcohol sensor 204 may be displayed on an external device that is connected to the alcohol concentration measurement apparatus 1. In this case, if the alcohol concentration measurement apparatus 1 is connected via the interface 208 to a PC (Personal Computer), which is the external device, the alcohol concentration measurement apparatus 1 transfers the usage start date and time, and the total usage time stored in the auxiliary storage device 207 to the PC. Further, the PC displays the extent of deterioration of the alcohol sensor 204 on the basis of the usage start date and time and the total usage time that are transferred. Moreover, the alcohol concentration measurement apparatus 1 may transfer a display image of the extent of deterioration generated by the deterioration level display control part 307 to the PC, and the PC may display the display image.

Furthermore, in the present embodiment, a case is described in which the extent of deterioration of the alcohol sensor based on the usage start date and time, and the extent of deterioration based on the total usage time are displayed on the display 102. However, it is in no way limited to this, and just one of the extents may be displayed.

In the present embodiment, the main storage device 206 and the auxiliary storage device 207 are examples of a non-transitory tangible medium. Furthermore, in the present embodiment, a case is described where the program is stored in the auxiliary storage device 207. However, it is in no way limited to this. For example, the program may be stored in a non-transitory tangible medium other than the auxiliary storage device 207. Other examples of non-transitory tangible media include a magnetic disk, an optomagnetic disk, a CD-ROM, a DVD-ROM, and a semiconductor memory that are connected via the interface 208. Furthermore, the program may be transmitted to the computer by a transmission line. In this case, the computer receiving the transmission transfers the program to the main storage device 206, and executes the processing described above.

Moreover, the program may be one that realizes a portion of the functions mentioned above.

Further, the program may be one that realizes the functions mentioned above by being combined with another program that has been previously stored in the auxiliary storage device 207, as a so-called difference file (difference program).

In one embodiment, a gas concentration measurement apparatus for performing a measurement of a gas concentration includes: a gas sensor configured to output an electrical signal corresponding to the gas concentration in breath; an exposure mechanism configured to expose the gas sensor to outside air when the gas concentration is measured; a measuring part configured to measure the gas concentration based on an electrical signal output by the gas sensor while the gas sensor is exposed to the outside air; and a notification part configured to perform a notification for replacement of the gas sensor in at least one of cases (a) where a total sum of value corresponding to a duration that the gas sensor has been exposed to the outside air exceeds a predetermined value and (b) where a total sum of value corresponding to a gas concentration measured by the measuring part exceeds a predetermined value.

In one embodiment, a gas concentration measurement apparatus for performing a measurement of a gas concentration includes: a gas sensor configured to output an electrical signal corresponding to the gas concentration; an exposure mechanism configured to expose the gas sensor to outside air when the gas concentration is measured; a measuring part configured to measure the gas concentration contained in breath of a test subject based on an electrical signal output by the gas sensor while the gas sensor is exposed to the outside air; and a notification part configured to perform a notification for replacement of the gas sensor when a total sum of a value corresponding to a duration that the gas sensor has been exposed to the outside air exceeds a predetermined value.

In the embodiments, the exposure mechanism can expose the gas sensor to the outside air for a duration that is monotonically non-decreasing with respect to the gas concentration.

In the embodiments, the exposure mechanism, after the measuring part measures the gas concentration, can finish exposure of the gas sensor once a duration that is monotonically non-decreasing with respect to the gas concentration measured by the measuring part elapses.

In the embodiments, the exposure mechanism can at least continue exposure of the gas sensor until a value of an electrical signal output by the gas sensor becomes less than or equal to a predetermined threshold; and the measuring part can perform a measurement of the gas concentration based on an integrated value of an electrical signal.

In the embodiments, the gas concentration measurement apparatus can further include a deterioration level display control part configured to display a fraction obtained by dividing a total sum of a value corresponding to a duration that the gas sensor has been exposed to the outside air by a predetermined value at which a notification for replacement of the gas sensor is performed.

In one embodiment, a gas concentration measurement apparatus for performing a measurement of a gas concentration includes: a gas sensor configured to output an electrical signal corresponding to the gas concentration; a measuring part configured to measure the gas concentration based on an electrical signal output by the gas sensor; and a notification part configured to perform a notification for replacement of the gas sensor when a total sum of a value corresponding to the gas concentration measured by the measuring part exceeds a predetermined value.

In the embodiments, the gas concentration measurement apparatus can further include a deterioration level display control part configured to display a fraction obtained by dividing a total sum of a value corresponding to a gas concentration measured by the measuring part by a predetermined value at which a notification for replacement of the gas sensor is performed.

In the embodiments, the notification part can perform a notification for replacement of the gas sensor when a usage time of the gas sensor exceeds a predetermined value.

In one embodiment, a replacement notification method of a gas sensor provided in a gas concentration measurement apparatus for performing a measurement of a gas concentration is provided, the method including: determining whether or not a total sum of a value corresponding to a duration that the gas sensor has been exposed to outside air exceeds a predetermined value; and performing a notification for replacement of the gas sensor when a total sum of a value corresponding to the duration that the gas sensor has been exposed to the outside air exceeds a predetermined value.

In one embodiment, a replacement notification method of a gas sensor provided in a gas concentration measurement apparatus for performing a measurement of a gas concentration is provided, the method including: measuring the gas concentration based on an electrical signal output by the gas sensor; determining whether or not a total sum of a value corresponding to the gas concentration measured by the measuring part exceeds a predetermined value; and performing a notification for replacement of the gas sensor when a total sum of a value corresponding to the gas concentration measured by the measuring part exceeds a predetermined value.

## Claims

1. A gas concentration measurement apparatus for performing a measurement of a gas concentration, the apparatus comprising:
a gas sensor configured to output an electrical signal corresponding to the gas concentration in breath;
a measuring part configured to measure the gas concentration based on an electrical signal output by the gas sensor; and
a notification part configured to perform a notification for replacement of the gas sensor in at least one of a case (a) where a total sum of value corresponding to a duration that the gas sensor has been exposed to outside air exceeds a predetermined value and a case (b) where a total sum of value corresponding to the gas concentration measured by the measuring part exceeds a predetermined value.

2. The gas concentration measurement apparatus according to Claim 1, further comprising:
an exposure mechanism configured to expose the gas sensor to the outside air for a duration that is monotonically non-decreasing with respect to the gas concentration.

3. The gas concentration measurement apparatus according to Claim 2,
wherein the exposure mechanism, after the measuring part measures the gas concentration, finishes exposure of the gas sensor once a duration that is monotonically non-decreasing with respect to the gas concentration measured by the measuring part elapses.

4. The gas concentration measurement apparatus according to Claim 2 or 3, wherein:
the exposure mechanism at least continues exposure of the gas sensor until a value of an electrical signal output by the gas sensor becomes less than or equal to a predetermined threshold; and
the measuring part performs a measurement of the gas concentration based on an integrated value of an electrical signal.

5. The gas concentration measurement apparatus according to any one of Claims 1 to 4, further comprising:
a deterioration level display control part configured to display a fraction obtained by dividing a total sum of a value corresponding to a duration that the gas sensor has been exposed to the outside air by a predetermined value at which a notification for replacement of the gas sensor is performed.

6. The gas concentration measurement apparatus according to any one of Claims 1 to 4, further comprising:
a deterioration level display control part configured to display a fraction obtained based on a table that associates the concentration and a recovery time, the recovery time being a time till the gas leaves the gas sensor.

7. The gas concentration measurement apparatus according to any one of Claims 1 to 4, further comprising
a deterioration level display control part configured to display a fraction obtained by dividing a total sum of a value corresponding to a gas concentration measured by the measuring part by a predetermined value at which a notification for replacement of the gas sensor is performed.

8. The gas concentration measurement apparatus according to any one of Claims 1 to 7,
wherein the notification part performs a notification for replacement of the gas sensor when a usage time of the gas sensor exceeds a predetermined value.

9. The gas concentration measurement apparatus according to any one of Claims 1 to 8,
wherein the gas sensor is configured to measure an alcohol concentration in breath.

10. The gas concentration measurement apparatus according to any one of Claims 1 to 8,
wherein the gas sensor is configured to measure at least one of a concentration of a gas in breath that indicates a body fat burn amount and a concentration of a gas in breath that causes oral odor.

11. A notification method of a gas concentration measurement apparatus,
wherein the gas concentration measurement apparatus comprises:
a gas sensor configured to output an electrical signal corresponding to a gas concentration in breath; and
a measuring part configured to measure the gas concentration based on the electrical signal from the gas sensor,
wherein the method comprises:
determining whether or not to perform a notification for replacement of the gas sensor; and
performing the notification for replacement of the gas sensor,
wherein the determining comprises at least one of (a) determining whether or not a total sum of value corresponding to a duration that the gas sensor has been exposed to outside air exceeds a predetermined value and (b) determining whether or not a total sum of value corresponding to the gas concentration measured by the measuring part exceeds a predetermined value,
and wherein the performing comprises performing a notification in at least one of a case (a) where a total sum of value corresponding to a duration that the gas sensor has been exposed to outside air exceeds a predetermined value and a case (b) where a total sum of value corresponding to the gas concentration measured by the measuring part exceeds a predetermined value.

12. The notification method according to Claim 11, further comprising:
exposing the gas sensor to the outside air for a monotonically non-decreasing time with respect to the gas concentration.

13. The notification method according to Claim 11 or 12, further comprising:
displaying a fraction obtained by dividing a total sum of value corresponding to a duration that the gas sensor has been exposed to the outside air by a predetermined value.

14. The notification method according to Claim 11 or 12, further comprising:
displaying a fraction obtained based on a table that associates the concentration and a recovery time, the recovery time being a time till the gas leaves the gas sensor.

15. The notification method according to any one of Claims 11 to 14, further comprising:
measuring at least one of (a) an alcohol concentration in breath, (b) a concentration of a gas in breath that indicates a body fat burn amount and (c) a concentration of a gas in breath that causes oral odor.
